# EUROPÄISCHE PATENTSCHRIFT

(11) **EP 0 736 295 B1**
(45) Veröffentlichungstag und Bekanntmachung des Hinweises auf die Patenterteilung: **24.07.2002**
(21) Anmeldenummer: 96810112.1
(22) Anmeldetag: 01.03.1996
(51) Int. Cl.: A61F 13/14, A61F 5/37

(54) **Schulter-Fixationsverband**
Bandage for shoulder fixation
Bandage pour la fixation de l'épaule

(30) Priorität: 04.04.1995 CH 94495
(43) Veröffentlichungstag der Anmeldung: 09.10.1996
(73) Patentinhaber: HOMED AG, 4712 Laupersdorf (CH)
(72) Erfinder: Hodel, Marianne, 4712 Laupersdorf (CH)

(56) Entgegenhaltungen:
- EP-A- 0 476 623
- EP-A- 0 576 901
- DE-C- 4 210 692
- DE-C- 4 314 785

## Beschreibung

Durch die DE-C-4314785 ist ein Schulter-Fixationsverband entsprechend dem Oberbegriff des Anspruchs 1 bekannt.
Ein Druck auf den Nacken soll erfindungsgemäss bei einem solchen Fixationsverband vermieden werden.

Der Fixationsverband gemäss Erfindung wird eingesetzt bei Oberarmfrakturen, Schulterluxationen und zur postoperativen Fixierung und weist die weiteren Merkmale des kennzeichnenden Teils des Anspruchs 1 auf.

Das Trägermaterial besteht aus 9 mm dicken und 8 cm breiten Polyurethan-Schaumstoff-Streifen, die auf einer Seite mit einem Polyamid-Stoff und auf der anderen Seite mit einem Baumwoll-Frotté-Stoff flammkaschiert werden, d. h. unter Wärmeeinwirkung untrennbar zusammengepresst werden. Diese Bandage wird ringsherum allseitig mit einem Schrägband (8) 40/20 mm eingefasst. Die Schulterkappe (1) wird aus demselben Material in eine der Schulter angepasste Form geschnitten und ermöglicht eine rutschfeste Fixation. Eine dauerelastische Schlauchbandage (2), welche oben und unten mit dem Polyurethan-Streifen zusammengenäht ist, dient zur Aufnahme des kranken Armes. Damit kein Druck auf den Nacken (3.1) entsteht, wird das Nacken-Trägerteil in der Mitte in zwei Teile (3.2) aufgeschlitzt. Diese beiden Teile werden um die gesunde Schulter wie ein Rucksack getragen, nach vorne um die Hand des kranken Armes geschlungen und auf Brusthöhe (5) mit einem Klett-Verschluss (6) befestigt. Das Taillen-Teil (4) beginnt unter der freiliegenden Hand des kranken Armes, wird um den Thorax bis zum kranken Arm im Ellbogen-Bereich geführt, um den Arm geschlungen und rückwärts auf dem Rücken mittels Klett-Verschluss (6) befestigt.

Damit keine Stoff-Unebenheiten im Bereich der Schulterkappe eintreten, werden hier zusätzlich je zwei Gummizüge (7) eingenäht. Durch Umkehr-Näherei von Frotté- und Polyamid-Oberfläche wird erreicht, dass sämtliche mit dem Körper in Berührung kommenden Teile aus Baumwoll-Frotté bestehen und die aussenliegenden Polyamid-Stoff-Teile der sicheren Befestigung durch Klett-Verschlüsse (6) dienen.

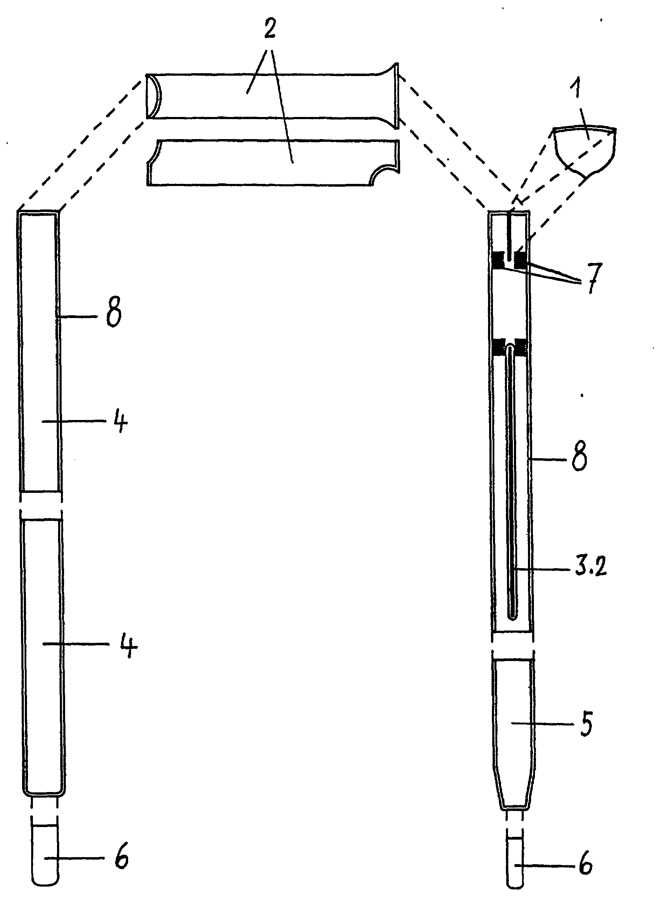

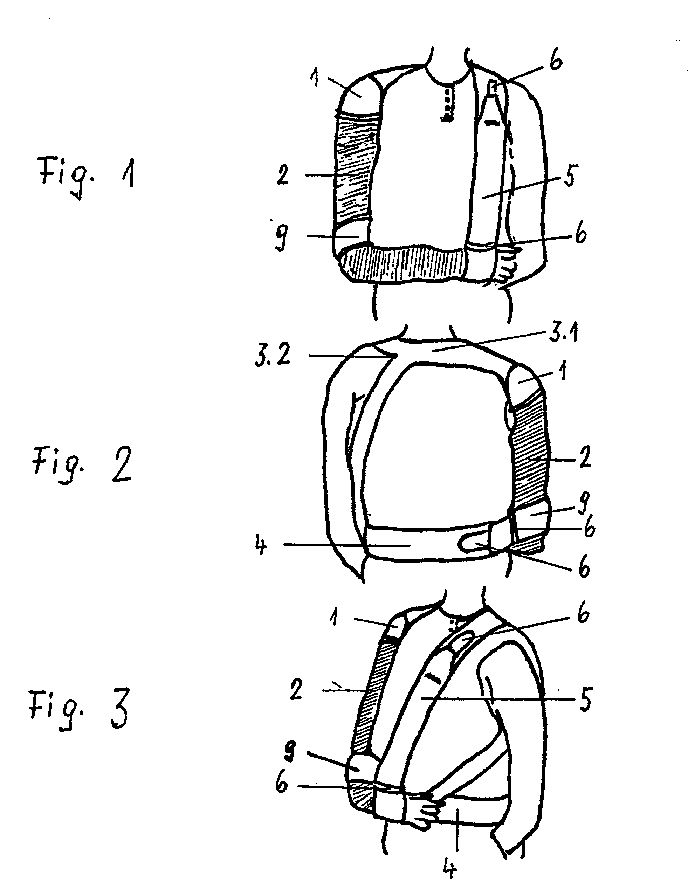

## Patentansprüche

1. Schulter-Fixationsverband zur Fixierung von Armfrakturen und Verletzungen des Schulter-Gürtels, bestehend aus einem Taillenteil (4), einem Nackenträgerteil, einer Schulterkappe (1) sowie einer dauerelastischen Schlauchbandage (2) für den Ober- und Unterarm, **dadurch gekennzeichnet, dass** das Nackenträgerteil in der Mitte in zwei Teile (3.2) aufgeschlitzt ist, um diese Teile wie einen Rucksack um die gesunde Schulter tragbar zu gestalten und dass der Schulter-Fixationsverband aus einem einteiligen, undehnbaren, 3-schichtigen Trägermaterial besteht, mit einer Innenschicht aus Baumwoll-Frottée-Stoff, einer Mittelschicht aus Polyurethan-Schaumstoff und einer Aussenschicht aus Polyamid-Stoff.

2. Schulter-Fixationsverband nach Anspruch 1, **dadurch gekennzeichnet, dass** im Bereich der Schulterkappe (1) vier Gummibänder (7) eingenäht sind, um eine straffe Führung des Verbandes zu gewährleisten.

## Claims

1. Immovable shoulder bandage used to fix fractures of the arm and shoulder girdle injuries, consisting of a waist section (4), a neck support section and a shoulder cap, **characterised in that** the neck support section is slit into two sections (3,2) in the middle, so as to enable these sections to be carried around the healthy shoulder like a haversack, and **in that** the immovable shoulder bandage consists of a single-section, non-extensible, 3-layer base material with an inner layer of cotton terry cloth, a middle layer of foamed polyurethane material, and an outer layer of polyamide material.

2. Immovable shoulder bandage in accordance with Claim 1, **characterised in that** four rubber strips (7) are sewn into the area of the shoulder cap (1) in order to ensure that the bandage is kept taut.

## Revendications

1. Bandage de fixation de l'épaule pour fractures de l'épaule et blessures de la ceinture scapulaire, comprenant une partie taille (4), une partie de soutien de la nuque, une calotte d'épaule, **caractérisé par le fait que** la partie de soutien de la nuque est fendue au centre (3.2) de façon à former deux parties de telle façon que ces parties puissent être supportées par l'épaule saine à la manière d'un sac à dos et **caractérisé par le fait que** le bandage de fixation de l'épaule est constitué d'un matériau support non extensible et d'une seule pièce comportant trois couches, une couche intérieure en coton éponge, une couche intermédiaire en polyuréthanne expansé et une couche extérieure en polyamide.

2. Bandage de fixation d'épaule selon la revendication 1, **caractérisé par le fait que** dans la zone de la calotte d'épaule (1), quatre bandes élastiques en caoutchouc (7) sont cousues pour assurer un guidage précis du bandage.
